# EUROPEAN PATENT APPLICATION

(11) **EP 3 165 241 A2**
(43) Date of publication of application: **10.05.2017**
(21) Application number: 16197076.9
(22) Date of filing: 03.11.2016
(51) Int. Cl.: A61M 1/00, A61M 5/168, A61M 39/08, A61M 39/28, F16K 7/06

(54) **ANTI-CLOGGING FLUID MANAGEMENT SYSTEM**

(30) Priority: 04.11.2015 US 201514932447
(71) Applicant: DePuy Mitek, LLC, Raynham, MA 02767 (US)
(72) Inventor: CHILDS, Joseph, Raynham, MA 02767 (US); THISTLE, Robert, Raynham, MA 02767 (US)
(74) Representative: Carpmaels & Ransford LLP

(57) **Abstract**

Various methods and devices for performing arthroscopic surgery are provided. In particular, methods and devices are provided for reshaping, unclogging, and vibrating tubing of an aspiration pump in a fluid management system. For example, a fluid management system is provided that includes a valve assembly disposed on a housing and configured to selectively pinch closed tubing disposed within a first pathway and a second pathway. The valve assembly is configured to bias tubing disposed within the first and second pathways to an open position after the tubing is pinched closed by the valve assembly. As another example, a fluid management system is provided that includes a vibratory mechanism coupled to a valve and configured to vibrate the valve so as to facilitate the flow of debris through tubing disposed within first and second tubing pathways.

## Description

### FIELD

The present invention relates generally to methods and devices for performing arthroscopic surgery, and in particular for preventing or removing clogs in an arthroscopic fluid management system.

### BACKGROUND

Minimally invasive surgery, also referred to herein as endoscopic surgery, often utilizes an irrigation system to force suitable biocompatible fluid into the area surrounding the surgical work site within a patient. The term "irrigation" is used broadly to mean any type of pressurized fluid flow whether it be for irrigation in particular or for other uses. Flexible plastic tubing is often used to transfer the fluid from a source to the work site and from the work site to a drain or other receptacle. Flexible tubing is also sometimes used as a pressure monitoring line to convey fluid pressure information to a control mechanism. Depending upon the procedure, the irrigating fluid is useful for various purposes such as tissue lavage, hydro-dissection, joint distension, uterine distension, etc. Known irrigation systems include electrically driven pump systems, in which a suitable fluid is pumped through flexible tubes from a source to the work site. Aspiration systems are also used and include a separate flexible tube that is connected to a vacuum source for aspirating the work site. Aspiration of the fluid can serve to either simply remove fluid to improve visibility, prevent undesirable fluid accumulation or high pressure at the work site, or to regulate the flow rate to maintain a predetermined fluid pressure at the work site.

Because the irrigation and aspiration functions are commonly used together, many combination irrigation/aspiration systems have been developed that perform both functions, often combined in one console which provides power and control. The irrigation system is generally used in conjunction with an aspiration system which removes the fluid pumped into the work site at a controlled rate depending on the flow rate selected by the surgeon. Each system utilizes a flexible tube to connect the fluid and vacuum sources to appropriate instruments inserted into the body.

While current systems can be effective to both irrigate and aspirate, the tubing can be prone to clogging. The flexible nature of the tubing can result in deformation of the tubing at a pinch point where flow through the tubing is controlled, and clogs can more readily occur due to such deformation. Large debris can also cause clogging to occur. Techniques are not readily available to clear clogs, and as a result the procedure can be prolonged increasing costs and resulting in user dissatisfaction.

Accordingly, there remains a need for improved methods and devices for performing arthroscopic surgery, and in particular for prevent or removing clogs in an arthroscopic fluid management system.

### SUMMARY

The present invention relates generally to methods and devices for performing arthroscopic surgery. In one aspect, a fluid management system is provided that includes a housing with an irrigation pump configured to couple to irrigation tubing for delivering fluid to a tissue site. The housing also includes an aspiration pump configured to couple to aspiration tubing for aspirating fluid from a tissue site. A coupling adjacent to the aspiration pump has first and second pathways formed therein that are configured to seat a first tubing portion and a second tubing portion of an aspiration tubing coupled to the aspiration pump. A valve assembly is disposed on the housing and is configured to selectively pinch closed tubing disposed within the first pathway and the second pathway. The valve assembly is configured to bias tubing disposed within the first and second pathways to an open position after the tubing is pinched closed by the valve assembly.

The fluid management system can vary in any number of ways. For example, the valve assembly can be slidably coupled to the coupling and can be slidable in a first direction to pinch closed tubing disposed within the first pathway and a second opposite direction to pinch closed tubing disposed within the second pathway. In another example, the valve assembly can include a tube guide configured to bias tubing disposed within one of the first and second pathways to an open position. As another example, the valve assembly can include first and second substantially concave cavities configured to seat tubing disposed within the first and second pathways, respectively, when the tubing is being biased to the open position. For another example, the valve assembly can include a cam loaded mechanism configured to provide a force on tubing seated in the first and second pathways to restore an original shape of the tubing when the tubing is in the open position. As yet another example, first and second biasing mechanisms can be coupled to the valve assembly with the first biasing mechanism being configured to bias tubing disposed within the first pathway to an open position and the second biasing mechanism being configured to bias tubing disposed within the second pathway to an open position.

In another aspect, a surgical method is provided that includes activating a fluid management device to deliver fluid through an irrigation tube to a tissue site and to aspirate fluid from the tissue site through a first aspiration tube. The method also includes activating a tool to cause a valve on the fluid management device to move from a first position, in which the first aspiration tube is open and a second aspiration tube is pinched closed, to a second position, in which the first aspiration tube is pinch closed and the second aspiration tube is open such that fluid is aspirated from the irrigation site through the second aspiration tube. In this method, the valve applies a force to the second aspiration tube to deform the second aspiration tube open when the valve moves from the first position to the second position.

The surgical method can vary in any number of ways. For example, the valve can slide to move from the first position to the second position. As another example, the valve can include a cam loaded mechanism that applies the force to the second aspiration tube to restore the second aspiration tube to an original shape of the second aspiration tube when the tube is in the open position. As another example, the valve can include a substantially concave cavity that seats the second aspiration tube when the tube is being deformed open.

In another aspect, a fluid management system is provided that includes a housing with an irrigation pump configured to couple to an irrigation tubing set. The housing also includes an aspiration pump configured to couple to an aspiration tubing set, a first tubing pathway for seating a first aspiration tube of an aspirating tubing set, and a second tubing pathway for seating a second aspiration tube of an aspiration tubing set. A selector valve is disposed on the housing and movable between a first position, a second position, and a neutral position. In the first position, the selector valve is configured to pinch a first aspiration tubing seated within the first tubing pathway to prevent the fluid flow therethrough while allowing the fluid flow through a second aspirating tubing seated within the second tubing pathway. In the second position, the selector valve is configured to pinch a second aspiration tubing seating within the second tubing pathway to prevent the fluid flow therethrough while allowing the fluid flow through a first aspirating tubing seating within the first tubing pathway. In the neutral position, the selector valve is configured to allow fluid flow through both a first aspiration tube seated within the first tubing pathway and a second aspiration tube seated within the second tubing pathway.

The fluid management system can vary in any number of ways. For example, the selector valve can be configured to rotate and pinch closed a length of tubing disposed within each of the first and second pathways. As another example, the selector valve can be rotatable 360° relative to the housing and the selector valve can be configured to sequentially squeeze a first tubing seated in the first tubing pathway and a second tubing seated within a second tubing pathway during a 360° rotation stroke. As yet another example, the housing can include an actuator for mechanically moving the selector valve between the first, second, and neutral positions. For another example, the system can include an irrigation tubing set having a tube with a first end portion coupled to the irrigation pump and a second end portion configured to couple to a tool, and an aspiration tubing set having a first tube coupled to the aspiration pump and second and third tubes each coupled to the first tube and seated within the first and second tubing pathways, respectively, with the second and third tubes each having an end configured to couple to a tool.

In another aspect, a surgical method is provided that includes activating a fluid management device to deliver fluid through an irrigation tube to a tissue site and to aspirate fluid from the tissue site through a first aspiration tube. The method also includes activating a tool to cause a valve on the fluid management device to pinch closed the first aspiration tube such that a second aspiration tube is opened and fluid is aspirated from the irrigation site through the second aspiration tube. The fluid management device rotates the valve along a length of at least one of the first and second aspirations tubes when a clog is detected.

The surgical method can vary in any number of ways. For example, prior to activating the fluid management device, the valve can be positioned in a neutral position such that the first and second aspiration tubes are open to allow fluid flow therethrough. As another example, the valve can rotate 360° when a clog is detected.

In another aspect, a fluid management system is provided that includes a housing with an irrigation pump configured to couple to irrigation tubing for delivering fluid to a tissue site. The housing also includes an aspiration pump configured to couple to aspiration tubing for aspirating fluid from a tissue site, a first tubing pathway for seating a first aspiration tube of an aspirating tubing set, and a second tubing pathway for seating a second aspiration tube of an aspiration tubing set. A valve is disposed on the housing and is configured to selectively occlude tubing disposed in one of the first and second tubing pathways while allowing fluid to flow through tubing disposed in the other one of the first and second tubing pathways. A vibratory mechanism is coupled to the valve and is configured to vibrate the valve so as to facilitate the flow of debris through tubing disposed within the first and second tubing pathways.

The fluid management system can vary in any number of ways. For example, the valve can be slidably coupled to the first tubing pathway and the second tubing pathway and can be slidable in a first direction to pinch closed tubing disposed within the first tubing pathway and a second opposite direction to pinch closed tubing disposed within the second tubing pathway. As another example, the valve can include a tube guide configured to bias tubing disposed within one of the first and second tubing pathways to an open position. In another example, the housing can have a self-cleaning mode that includes vibration of the vibratory mechanism. In another embodiment, the valve can include a cam loaded mechanism configured to provide a force on tubing seated in the first and second tubing pathways to restore an original shape of the tubing when the tubing is in the open position.

In another aspect, a surgical method is provided that includes activating a fluid management device to deliver fluid through an irrigation tube to a tissue site and to aspirate fluid from the tissue site through a first aspiration tube. The method also includes activating a tool to cause a valve on the fluid management device to move from a first position, in which the first aspiration tube is open and a second aspiration tube is closed, to a second position, in which the first aspiration tube is pinch closed and the second aspiration tube is open such that fluid is aspirated from the irrigation site through the second aspiration tube. In the method, at least one of a user or the fluid management devices activates a vibratory mechanism coupled to the valve to cause the valve to vibrate and thereby aid in the passage of debris through the first and second aspirations tubes.

The surgical method can vary in any number of ways. For example, prior to activating the vibratory mechanism, the valve can be positioned in a neutral position such that the first and second aspiration tubes are open to allow fluid flow therethrough. The method can include the step of, prior to activating the vibratory mechanism, activating a self-cleaning mode such that the valve moves between the first position and the second position.

### BRIEF DESCRIPTION OF DRAWINGS

This invention will be more fully understood from the following detailed description taken in conjunction with the accompanying drawings, in which:
FIG. 1 is a side view of an embodiment of a housing with an irrigation mechanism and an aspiration pump;
FIG. 2 is a top-down view of an embodiment of a valve assembly;
FIG. 3 is a top-down view of the valve assembly of FIG. 2;
FIG. 4 is another top-down view of the valve assembly of FIG. 2;
FIG. 5 is a top-down view of another embodiment of a valve assembly with a guard removed;
FIG. 6 is a partial top-down view of the valve assembly of FIG. 5 with a guard added;
FIG. 7 is a perspective exploded-view of the valve assembly of FIG. 5;
FIG. 8 is a top-down view of another embodiment of a valve assembly with a guard removed;
FIG. 9 is a partial top-down view of the valve assembly of FIG. 8 with a guard added;
FIG. 10 is a perspective exploded-view of the valve assembly of FIG. 8;
FIG. 11 is a front view of another embodiment of a selector valve;
FIG. 12 is a front view of the selector valve of FIG. 11;
FIG. 13 is another front view of the selector valve of FIG. 11; and
FIG. 14 is a top-down view of an embodiment of a selector valve having a vibratory mechanism.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

Various methods and devices for performing arthroscopic surgery are provided. In particular, methods and devices are provided for reshaping, unclogging, and vibrating tubing of an aspiration pump in a fluid management system.

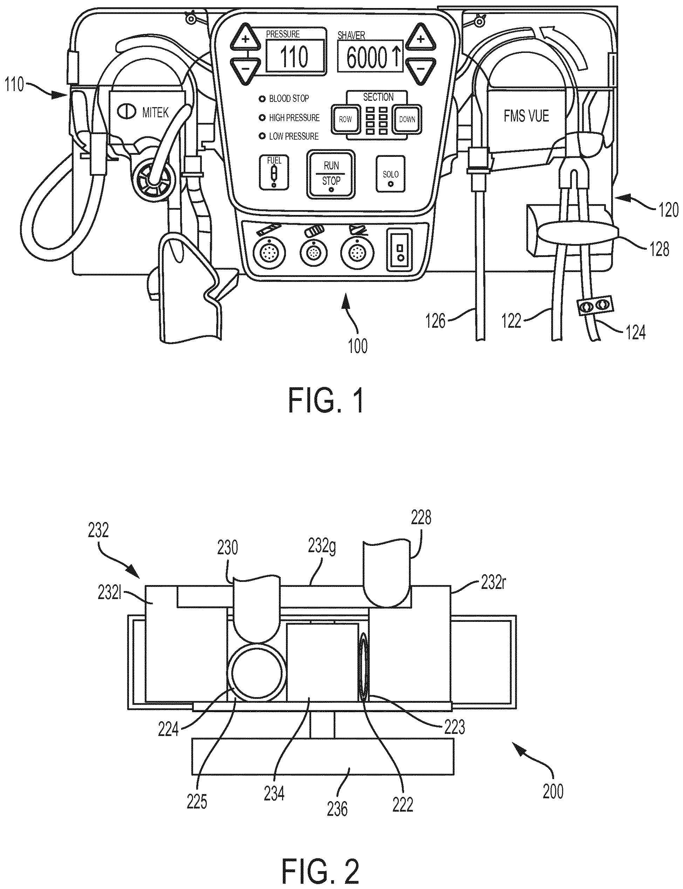

Fluid management systems are used during minimally invasive surgery to irrigate and aspirate an area surrounding a surgical work site within a patient. As seen in FIG. 1, a single device 100 is provided that includes both an irrigation mechanism 110 that can deliver fluid to a work site and an aspiration pump 120 that can remove fluid and tissue from the work site. The aspiration pump 120 can remove fluid and tissue from the work site through two suction tubes. For example, the device 100 can have a cannula suction tube 124 that can remove fluid from a cannula at the work site and a tool suction tube 122 that can connect to a tool or instrument being used, such as a shaver, that can remove fluid as the tool is operated. Both tubes 122, 124 can deliver fluid and any tissue to a waste bag through waste tube 126. Suction can be delivered through only one of the two tubes 122, 124 at a time by pinching off one of the two tubes 122, 124 at a pinch valve 128. This allows a user to switch between suction through the cannula and suction through the tool. Pinching the tubes 122, 124, however, may lead to deformation and clogging of the tubes 122, 124 at a point where the pinch valve 128 pinches each of the tubes 122, 124. If true, the pinch valve 128 defaults to a position in which one tube remains open and another tube is closed, for example the cannula suction tube 124 open and the tool suction tube 122 closed, deformation and clogging may be exacerbated in the tube that is closed for a prolonged period.

A fluid management system can thus be provided with a housing that includes an aspiration pump configured to couple to aspiration tubing for aspirating fluid from a tissue site. The aspiration pump can be adjacent to a coupling that has first and second pathways formed therein that are configured to seat a first tubing portion and a second tubing portion of an aspiration tubing coupled to the aspiration pump. The housing can also include a valve assembly that is configured to selectively pinch closed tubing disposed within one of the first pathway and the second pathway while also being configured to bias tubing disposed within the other one of the first and second pathways to an open position after the tubing is pinch closed by the valve assembly. Biasing the tubing to an open position can cause the tubing to re-conform to an open position after the tubing has been pinched to a closed position, and may prevent deformation and clogging of the tubing during use and over time.

FIGS. 2-4 illustrate one embodiment of a valve assembly 200 having a biasing mechanism that can prevent deformation and clogging of aspiration tube. The valve assembly can replace the pinch valve 128 of FIG. 1. The valve assembly 200 has a first pathway 223 that is shown having a first suction tube 222 disposed therethrough and a second pathway 225 that is shown having a second suction tube 224 disposed therethrough. A pincher 232 slides in tandem with a first plunger 228 and a second plunger 230. The first plunger 228 and the second plunger 230 are configured to engage the first suction tube 222 and the second suction tube 224, respectively. The pincher 232 includes a left side portion 232*l* and a right side portion 232r positioned on either side of the first and second pathways 223, 225, as well as a groove 232g that receives the first and second plungers 228, 230. An anvil 234 is disposed between the first pathway 223 and the second pathway 225, and is coupled to a handle 236. The pincher 232 is slidable in a left or a right direction, as can be seen in FIGS. 2-4.

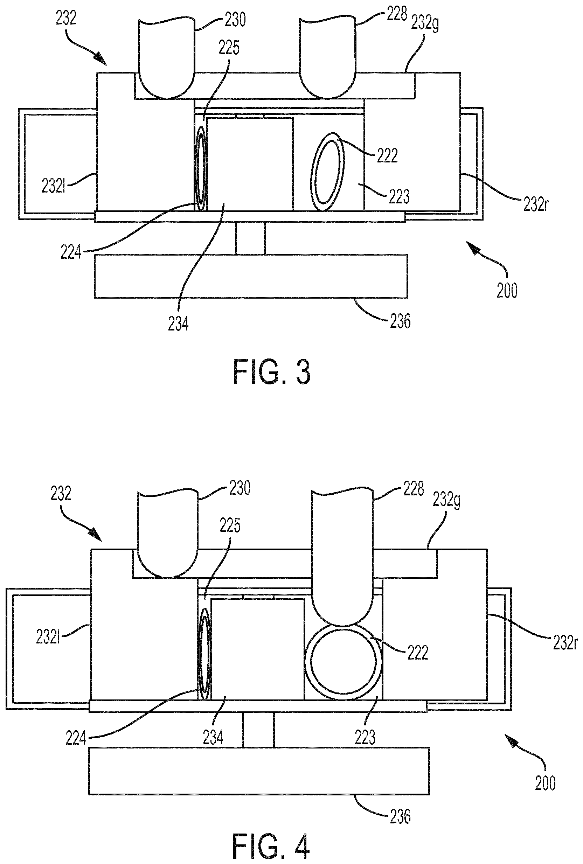

As the pincher 232 slides in a right direction, the left side portion 232*l* contacts and pinches closed the second suction tube 224 as the second suction tube 224 is pinched between the anvil 234 and the left side portion 232*l*. At the same time, the second plunger 230 is forced to retract out of the second pathway 225 into the groove 232g and out of contact with the second suction tube 224. The first plunger 228 advances into the first pathway 223. As can be seen in FIG. 3, as the pincher 232 continues to move right, the second suction tube 224 is pinched closed, but the first suction tube 222 can be deformed from previously being pinched between the anvil 234 and the right side portion 232r of the pincher 232. The first plunger 228 advances into engagement with the first suction tube 222 as the first plunger 228 enters the first pathway 223. As seen in FIG. 4, engagement between the first plunger 228 and the first suction tube 222 will bias the first suction tube 222 to an open position. The first suction tube 222 can open to a position in which the first suction tube 222 contacts the anvil 234, the right side portion 232r, a front of the valve assembly 200, and the first plunger 228. The first suction tube 222 can thus be entirely re-conformed to a pre-pinching position, e.g. to have a circular cross-sectional shaft, preventing deformation and clogging of the first suction tube 222. The pincher 232 of the valve assembly 200 is also configured to slide left, which would reverse the process described above and pinch the first suction tube 222 closed while the second plunger 230 engage with and forces the second suction tube 224 to an open configuration.

The plungers 228, 230 can be moved in a variety of ways. For example, the plungers 228, 230 can be mechanically moved through operation of a motor. A system processor can control movement of the plungers. A mechanical design controlled by software can be used.

In another embodiment, the device can include a self-cleaning mode, in which the valve assembly 200 is activated and cycles through opening and closing each of the tubes in the first and second pathways. The mode can be user activated and can use a mechanical design controlled by software.

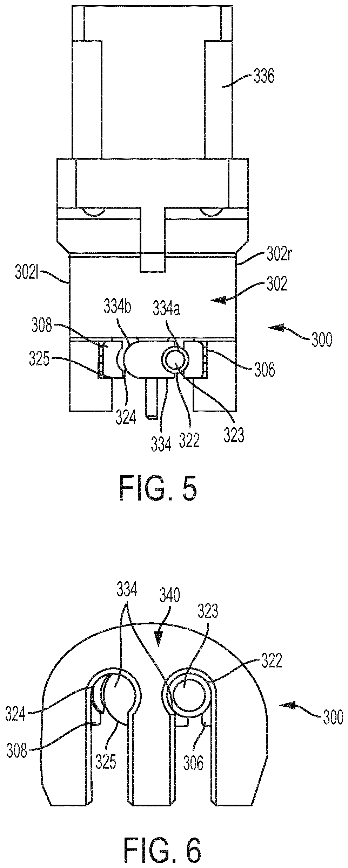

FIGS. 5-7 illustrate another embodiment of a valve assembly 300 having a biasing mechanism that can prevent deformation and clogging of aspiration tube. The valve assembly can replace the pinch valve 128 of FIG. 1. The valve assembly 300 has a case 302 that includes a first shoe 306 and a second shoe 308 that define a first pathway 323 that is shown having a first suction tube 322 disposed therethrough and a second pathway 325 that is shown having a second suction tube 324 disposed therethrough. The shoes 306, 308 are each concave surfaces that conform to the cylindrical shape of the tubes 322, 324. A left spring 310 and a right spring 312 are seated within openings on a left side portion 302*l* of the case 302 and a right side portion 302r of the case 302. Panels 314 and arms 315 keep the springs 310, 312 engaged with the shoes 306, 308 so that the shoes 306, 308 are biased towards the first pathway 323 and the second pathway 325. A cam mechanism 334 is rotatably disposed between the first pathway 323 and the second pathway 325, and is coupled to a motor arm 336a of a rotary motor 336. The cam mechanism 334 has a concave side 334a and a convex side 334b and is configured to rotate about a longitudinal axis L of the motor arm 336a. The motor 336 is coupled to the case 302 with screws 342, and a guard 340 encapsulates the case 302.

As the cam mechanism 334 rotates about axis L, the cam mechanism 334 moves to an orientation where the cam mechanism 334 is horizontal with respect to the tubes 322, 324 and the concave side 334a and the convex side 334b each engage a tube. As seen in FIGS. 5 and 6, when the cam mechanism 334 is horizontal, the concave side 334a of the cam mechanism 334 contours and seats the concave curvature of the first shoe 306. The first shoe 306 is biased into the first pathway 323 so that the concave side 334a of the cam mechanism 334 and the first shoe 306 engage the first suction tube 322. Because the concave side 334a of the cam mechanism 334 and the first shoe 306 form an open, cylindrical space, the first suction tube 322 is forced into an open position. The first suction tube 322 can thus be entirely re-conformed to a pre-pinching open position, preventing deformation and clogging of the first suction tube 322. At the same time, the convex side 334b of the cam mechanism 334 fills the concave curvature of the second shoe 308. The second shoe 308 is biased into the second pathway 325 so that the convex side 334b of the cam mechanism 334 and the second shoe 308 engage the second suction tube 324 therebetween. Because the convex side 334b of the cam mechanism 334 conforms to the concave curvature of the second shoe 308, the second suction tube 324 is pinched closed therebetween. The first suction tube 322 can thus be entirely re-conformed to a pre-pinching position, preventing deformation and clogging of the first suction tube 322, while the second suction tube 324 is pinched closed. The cam mechanism 334 can continue to rotate, bringing the convex side 334b to pinch closed the first suction tube 322 and bringing the concave size 334a to re-conform the second suction tube 324 to a pre-pinching position and to thereby prevent deformation and clogging of the second suction tube 324.

As explained above with respect to the previous embodiment, the device can also have a self-cleaning mode. The mode can be user activated and can use a mechanical design controlled by software.

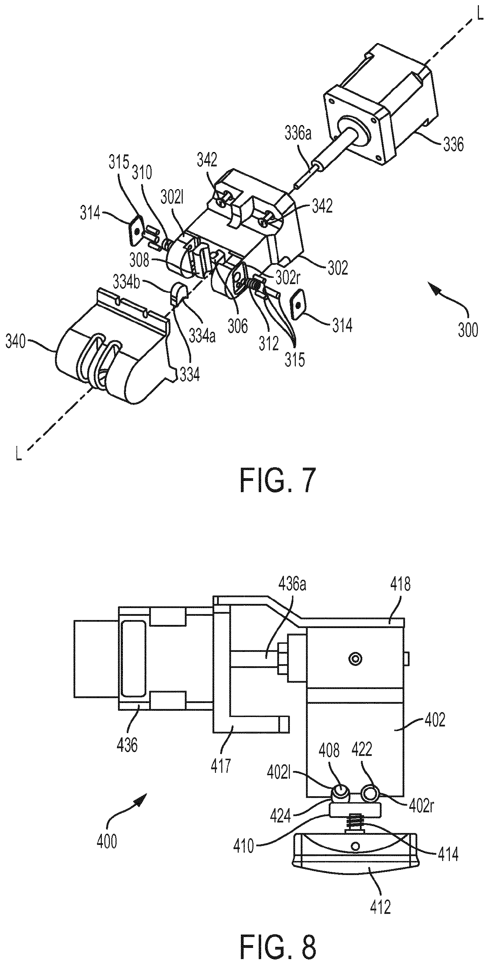

FIGS. 8-10 illustrate another embodiment of a valve assembly 400 having a biasing mechanism that can prevent deformation and clogging of aspiration tube. The valve assembly can replace the pinch valve 128 of FIG. 1. The valve assembly 400 has a case 402 that includes a first plunger 406 that retracts out of and advances into a first pathway 423 that seats a first suction tube 422. The case 402 also includes a second plunger 408 that retracts out of and advances into a second pathway 425 that seats a second suction tube 424. The first plunger 406 has a head 406h that is configured to engage the first suction tube 422, a spring 406s configured to bias the first plunger 406 out of engagement with the first suction tube 422, and an angled end 406a opposite to the head 406h. The second plunger 408 has a head 408h that is configured to engage the second suction tube 424, a spring 408s configured to bias the second plunger 408 out of engagement with the second suction tube 424, and an angled end 408a opposite to the head 408h. The first plunger 406 sits in a first channel C1 that aligns with a first lumen L1 that extends from the first channel C1 to the first pathway 422. The second plunger 408 sits in a second channel C2 that aligns with a second lumen L2 that extends from the second channel C2 to the second pathway 424. A rear wall 410 is rotatably coupled to a handle 412 and is biased towards the case 402 by a spring 414. The rear wall 410 is configured to engage the tubes 422, 424 to force the tubes 422, 424 into cavities 402*l*, 402r on a front side of the case 402, which cavities 402*l*, 402r have a re-conforming geometry in a shape of a non-deformed tube, e.g. a cylindrical shape. Cover 416 couples to the case 402 and encloses the plungers 406, 408. A cam mechanism 434 is linearly disposed between the angled end 406a of the first plunger 406 and the angled end 408a of the second plunger 408, and is coupled to a motor arm 436a of a linear motor 436. The cam mechanism 434 has a triangular top 434t that is configured to engage the angled ends 406a, 408a of the plungers 406, 408 to drive the ends 406a, 408a towards the pathways 423, 425, respectively. The cam mechanism 434 is configured to move right and left along a longitudinal axis L3 of the motor arm 436a. A side panel 417 and a back panel 418 couple the motor 436 to the case 402 while leaving space to allow for linear action of the motor arm 436a, as can be seen in FIG. 8. A guard 440 encapsulates the front of the case 402.

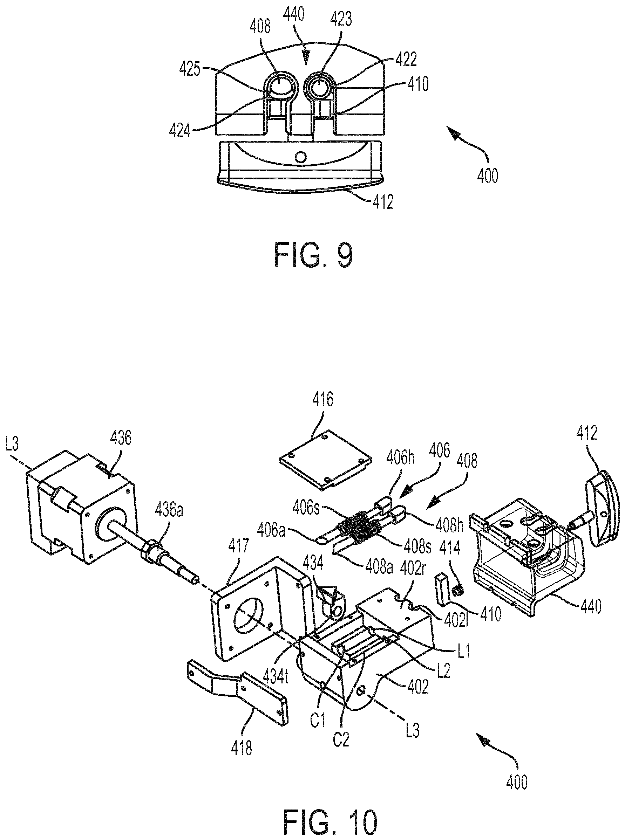

As the cam mechanism 434 moves linearly back and forth along axis L3 due to action of the motor 436, the triangular top 434t of the cam mechanism 434 engages each of the angled ends 406a, 408a of the plungers 406, 408 in turn to drive the plungers 406, 408 towards the pathways 423, 425, respectively. For example, as the cam mechanism 434 moves towards the second plunger 408 as seen in FIGS. 8 and 9, the cam mechanism 434 can engage and force the second plunger 408 forward. As the spring bias of the spring 408s is overcome, the head 408h of the second plunger 408 enters the cavity 402*l* and contacts the second suction tube 424. As the head 408h continues into the cavity 402*l*, the second suction tube 424 is pinched and deformed between the head 408h and the rear wall 410. At the same time, the triangular top 434t of the cam mechanism 434 is no longer applying force to the first plunger 406, and the spring 406s biases the first plunger 406 away from the cavity 402r. The rear wall 410 continues to engage with the first suction tube 422 as the first plunger 406 retracts away from the first suction tube 422, and the rear wall 410 forces the first suction tube 422 into the cavity 402r. The first suction tube 422 is forced into engagement with the re-conforming geometry of the cavity 402r and can re-conform to a pre-pinching position, preventing deformation and clogging of the first suction tube 422, while the second suction tube 424 is pinched closed. The cam mechanism 434 can then move towards the first plunger 406 along the axis L3, bringing the triangular top 434t of the cam mechanism 434 into contact with the first plunger 406 and forcing the first plunger 406 forward into pathway 423 to pinch closed the first suction tube 422 and allowing the second plunger 408 to retract. The rear wall 410 then forces the second suction tube into the cavity 402*l*, which re-conforms the second suction tube 424 to a pre-pinching position and prevents deformation and clogging of the second suction tube 424.

As explained above with respect to the previous embodiments, the device can also have a self-cleaning mode. The mode can be user activated and can use a mechanical design controlled by software.

A selector valve may alternatively be used to prevent deformation and clogging of a tube that is pinched closed. For example, a housing can have an aspiration pump configured to couple to an aspiration tubing set, a first tubing pathway for seating a first aspiration tube of an aspirating tubing set, and a second tubing pathway for seating a second aspiration tube of an aspiration tubing set. A selector valve can be disposed on the housing and movable between multiple positions. The selector valve can have a neutral position in which the selector valve is configured to allow fluid flow through both the first aspiration tube seated within the first tubing pathway and the second aspiration tube seated within the second tubing pathway. The selector valve can also have a first position, in which the selector valve is configured to pinch the first aspiration tubing seated within the first tubing pathway to prevent the fluid flow therethrough while allowing the fluid flow through the second aspirating tubing seated within the second tubing pathway. The selector valve can further have a second position, in which the selector valve is configured to pinch the second aspiration tubing seating within the second tubing pathway to prevent the fluid flow therethrough while allowing the fluid flow through the first aspirating tubing seating within the first tubing pathway.

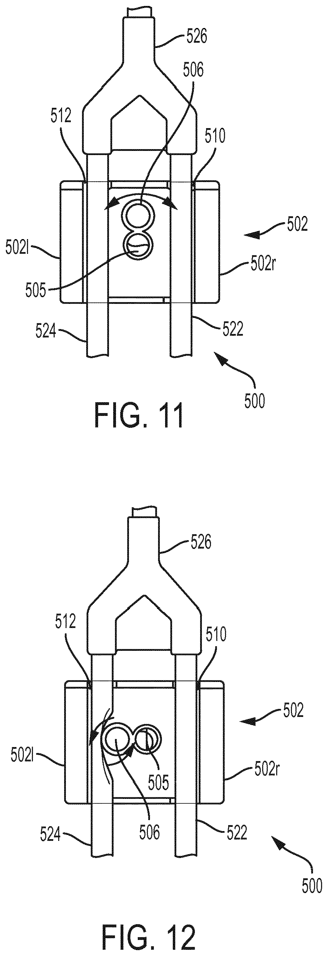

FIGS. 11-13 illustrate an embodiment of a selector valve 500 having a biasing mechanism that can prevent deformation and clogging of aspiration tube. The valve assembly can replace the pinch valve 128 of FIG. 1. The selector valve 500 has a case 502 that includes a rotating arm 504 with a roller 506 rotatably disposed on an end of the rotating arm 504. The rotating arm 504 is coupled at a central point 505 on the case 502 to a rotary motor (not shown) that is configured to rotate the rotating arm in a 360° motion about the central point 505. The case 502 forms flat walls 502r, 502*l* on each side of the rotating arm 504 and the roller 506. A first tubing pathway 510 is formed along the flat wall 502r and is configured to seat a first aspiration tube 522 of an aspirating tubing set 526. A second tubing pathway 512 is formed along the flat wall 502*l* and is configured to seat a second aspiration tube 524 of the aspirating tubing set 526. The roller 506 is configured to rotate into the first and second tubing pathways 510, 512 and to engage the tubes 522, 524.

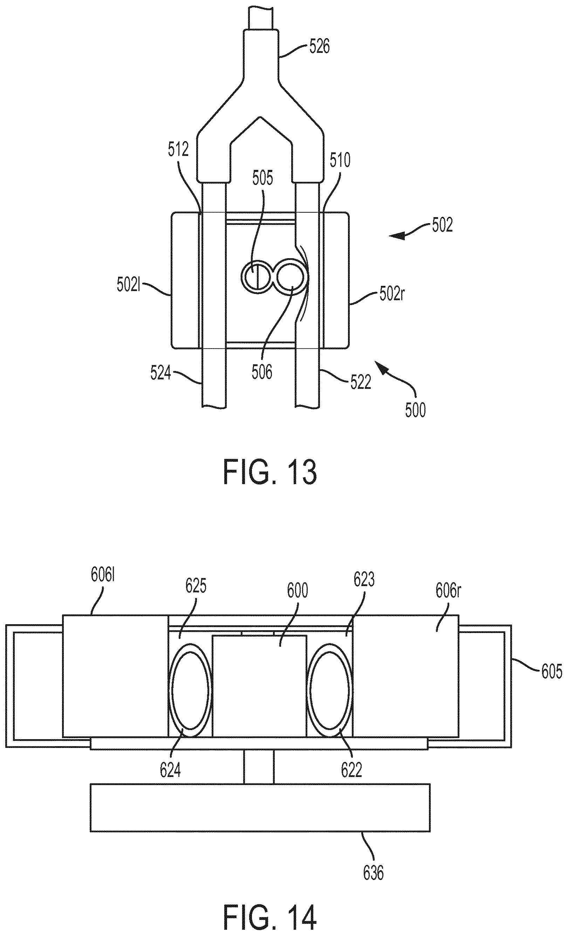

As the rotating arm 504 rotates the roller 506 about the central point 505, the roller 506 can be disposed at a neutral position between the tubes 522, 524, as shown in FIG. 11. At this point, the selector valve 500 allows fluid flow through both the first aspiration tube 522 and the second aspiration tube 524. As the rotating arm 504 continues to rotate as seen in FIG. 12, the rotating arm 504 rotates the roller 506 into the first tubing pathway 510 and into engagement with the first aspiration tube 522. The roller 506 rolls along a length of the first aspiration tube 522 until the roller 506 presses firmly against the flat wall 502r, and the first aspiration tube 524 is deformed and pinched between the roller 506 and the flat wall 502r. At this point, the selector valve 500 is in a first position, in which the selector valve 500 pinches the first aspiration tubing 522 to prevent fluid flow therethrough while allowing fluid flow through the second aspirating tubing 524. As the rotating arm 504 continues to rotate as seen in FIG. 13, the rotating arm 504 rotates the roller 506 into the second tubing pathway 512 and into engagement with the second aspiration tube 524. The roller 506 rolls along a length of the second aspiration tube 524 until the roller 506 presses firmly against the flat wall 502*l*, and the second aspiration tube 524 is pinched between the roller 506 and the flat wall 502*l*. At this point, the selector valve 500 is in a second position, in which the selector valve 500 pinches the second aspiration tubing 524 to prevent fluid flow therethrough while allowing fluid flow through the first aspirating tubing 522.

The roller 506 can be stopped at each position, or the rotating arm 504 can rotate the roller 506 around in 360° motions for one or more cycles. A user can activate a predetermined number of cycles, causing a peristaltic effect in the tubes 522, 524 and forcing any clogged material or fluids through the tubes 522, 524. Both the roller 506 and the rotating arm 504 can rotate in either direction.

As explained above with respect to the previous embodiments, the device can also have a self-cleaning mode. A direction of the roller can be selected to push any trapped tissue up toward a waste collection bag. The mode can be user activated and can use a mechanical design controlled by software.

In another embodiment, a vibratory mechanism can be coupled to a valve configured to selectively pinch tubing closed to prevent deformation and clogging of the tubing. A housing can include an aspiration pump configured to couple to aspiration tubing, a first tubing pathway for seating a first aspiration tube of an aspirating tubing set, and a second tubing pathway for seating a second aspiration tube of the aspiration tubing set. The housing can also include the valve disposed on the housing and configured to selectively pinch tubing disposed in one of the first and second tubing pathways while allowing fluid to flow through tubing disposed in the other one of the first and second tubing pathways. The valve can include any of the valves from FIGS. 1-13 or any currently known in the art. The vibratory mechanism coupled to the valve can be configured to vibrate the valve and/or the tubing so as to facilitate the flow of debris through tubing.

FIG. 14 illustrates an embodiment of a vibratory mechanism 600 that can couple to a valve on an aspiration pump, for example coupling to the pinch valve 128 of FIG. 1. A valve 602 has a case 605, a first pathway 623 having a first suction tube 622 disposed therethrough, and a second pathway 625 having a second suction tube 624 disposed therethrough. A pincher 606 includes a left side portion 606*l* and a right side portion 606r positioned on either side of the first and second pathways 223, 225. The vibrating mechanism 600 is disposed between the first pathway 623 and the second pathway 625, and is coupled to a handle 636 on a front of the valve 602 and a motor (not shown) on a back of the valve 602. The pincher 606 is configured to be slidable in a left or a right direction, and engages each tube 622, 624 in turn as the pincher 606 slides right and left. The tubes 622, 624 are pinched, in turn, between the pincher 602 and the vibrating mechanism 600.

The pincher can also slide to a neutral position, as shown in FIG. 14, with each tube 622, 624 partially open. At this point, the vibrating mechanism can vibrate, causing vibration of the tubes 622, 624 to release any clogs that may have formed in the tubes 622, 624.

While the vibrating mechanism 600 in FIG. 14 is shown coupled to valve 602, in other variations the vibratory mechanism can be used in combination with any of the valves described above. Additionally the vibrating mechanism can be incorporated into the cam mechanisms of the valves described above, or the vibrating mechanism can be a standalone unit. The vibrating mechanism 600 can in other embodiments be activated as part of a self-cleaning mode on a housing with an aspiration pump. The mode can be user activated and can use a mechanical design controlled by software.

The devices disclosed herein can also be designed to be disposed of after a single use, or they can be designed to be used multiple times, particularly the tubing. In either case, however, the device can be reconditioned for reuse after at least one use. Reconditioning can include any combination of the steps of disassembly of the device, followed by cleaning or replacement of particular pieces and subsequent reassembly. In particular, the device can be disassembled, and any number of the particular pieces or parts of the device can be selectively replaced or removed in any combination, especially the tubing. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, components of the invention described herein will be processed before use. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

Typically, the device is sterilized, particularly the tubing. This can be done by any number of ways known to those skilled in the art including beta or gamma radiation, ethylene oxide, steam, and a liquid bath (e.g., cold soak). An exemplary embodiment of sterilizing a device including internal circuitry is described in more detail in U.S. Pat. Pub. No. 2009/0202387 filed February 8, 2008 and entitled "System And Method Of Sterilizing An Implantable Medical Device." It is preferred that device, if implanted, is hermetically sealed. This can be done by any number of ways known to those skilled in the art.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A fluid management system, comprising:
a housing having
an irrigation pump configured to couple to irrigation tubing for delivering fluid to a tissue site,
an aspiration pump configured to couple to aspiration tubing for aspirating fluid from a tissue site,
a coupling adjacent to the aspiration pump and having first and second pathways formed therein that are configured to seat a first tubing portion and a second tubing portion of an aspiration tubing coupled to the aspiration pump, and
a valve assembly disposed on the housing and configured to selectively pinch closed tubing disposed within the first pathway and the second pathway, the valve assembly being configured to bias tubing disposed within the first and second pathways to an open position after the tubing is pinched closed by the valve assembly.

2. The system of claim 1, wherein the valve assembly is slidably coupled to the coupling and is slidable in a first direction to pinch closed tubing disposed within the first pathway and a second opposite direction to pinch closed tubing disposed within the second pathway.

3. The system of claim 1 or 2, wherein the valve assembly includes a tube guide configured to bias tubing disposed within one of the first and second pathways to an open position.

4. The system of claim 1, 2 or 3, wherein the valve assembly includes first and second substantially concave cavities configured to seat tubing disposed within the first and second pathways, respectively, when the tubing is being biased to the open position.

5. The system of claim 1, 2, 3 or 4, wherein the valve assembly includes a cam loaded mechanism configured to provide a force on tubing seated in the first and second pathways to restore an original shape of the tubing when the tubing is in the open position.

6. The system of claim 1, 2, 3, 4 or 5, further comprising first and second biasing mechanisms coupled to the valve assembly, the first biasing mechanism being configured to bias tubing disposed within the first pathway to an open position, and the second biasing mechanism being configured to bias tubing disposed within the second pathway to an open position.

7. A fluid management system, comprising:
a housing having
an irrigation pump configured to couple to an irrigation tubing set,
an aspiration pump configured to couple to an aspiration tubing set, and
a first tubing pathway for seating a first aspiration tube of an aspirating tubing set, and a second tubing pathway for seating a second aspiration tube of an aspiration tubing set; and
a selector valve disposed on the housing and movable between
a first position, in which the selector valve is configured to pinch a first aspiration tubing seated within the first tubing pathway to prevent the fluid flow therethrough while allowing the fluid flow through a second aspirating tubing seated within the second tubing pathway,
a second position, in which the selector valve is configured to pinch a second aspiration tubing seating within the second tubing pathway to prevent the fluid flow therethrough while allowing the fluid flow through a first aspirating tubing seating within the first tubing pathway, and
a neutral position in which the selector valve is configured to allow fluid flow through both a first aspiration tube seated within the first tubing pathway and a second aspiration tube seated within the second tubing pathway.

8. The system of claim 7, wherein the selector valve is configured to rotate and pinch closed a length of tubing disposed within each of the first and second pathways.

9. The system of claim 7 or 8, wherein the selector valve is rotatable 360° relative to the housing and the selector valve is configured to sequentially squeeze a first tubing seated in the first tubing pathway and a second tubing seated within a second tubing pathway during a 360° rotation stroke.

10. The system of claim 7, 8 or 9, wherein the housing includes an actuator for mechanically moving the selector valve between the first, second, and neutral positions.

11. The system of claim 7, 8, 9 or 10, further comprising an irrigation tubing set having a tube with a first end portion coupled to the irrigation pump and a second end portion configured to couple to a tool, and an aspiration tubing set having a first tube coupled to the aspiration pump and second and third tubes each coupled to the first tube and seated within the first and second tubing pathways, respectively, the second and third tubes each having an end configured to couple to a tool.

12. A fluid management system, comprising:
a housing having
an irrigation pump configured to couple to irrigation tubing for delivering fluid to a tissue site,
an aspiration pump configured to couple to aspiration tubing for aspirating fluid from a tissue site, and
a first tubing pathway for seating a first aspiration tube of an aspirating tubing set, and a second tubing pathway for seating a second aspiration tube of an aspiration tubing set;
a valve disposed on the housing and configured to selectively occlude tubing disposed in one of the first and second tubing pathways while allowing fluid to flow through tubing disposed in the other one of the first and second tubing pathways; and
a vibratory mechanism coupled to the valve and configured to vibrate the valve so as to facilitate the flow of debris through tubing disposed within the first and second tubing pathways.

13. The system of claim 12, wherein the valve is slidably coupled to the first tubing pathway and the second tubing pathway and is slidable in a first direction to pinch closed tubing disposed within the first tubing pathway and a second opposite direction to pinch closed tubing disposed within the second tubing pathway.

14. The system of claim 12 or 13, wherein the valve includes a tube guide configured to bias tubing disposed within one of the first and second tubing pathways to an open position.

15. The system of claim 12, 13 or 14, wherein the housing has a self-cleaning mode that includes vibration of the vibratory mechanism.

16. The system of claim 12, 13, 14 or 15, wherein the valve includes a cam loaded mechanism configured to provide a force on tubing seated in the first and second tubing pathways to restore an original shape of the tubing when the tubing is in the open position.
